# EUROPEAN PATENT SPECIFICATION

(11) **EP 1 281 396 B1**
(45) Date of publication and mention of the grant of the patent: **18.05.2011**
(21) Application number: 02292849.3
(22) Date of filing: 15.11.2002
(51) Int. Cl.: A61L 27/60

(54) **METHOD FOR PRODUCING ARTIFICIAL SKIN**
VERFAHREN ZUR HERSTELLUNG VON KUNSTHAUT
PROCÉDÉ DE FABRICATION DE PEAU ARTIFICIELLE

(30) Priority: 18.06.2002 JP 2002177601; 06.11.2002 JP 2002323030
(43) Date of publication of application: 05.02.2003
(60) Divisional application: 10012105.2
(73) Proprietor: SHISEIDO COMPANY, LTD., Tokyo 104-8010 (JP)
(72) Inventor: Amano Satoshi, Shiseido Res. Ctr. Kanazama Hakkei, Yokohama-shi, Kanagawa 236-0004 (JP); Ogura, Yuki, Shiseido Res. Ctr. Kanazama Hakkei, Yokohama-shi, Kanagawa 236-0004 (JP); Matsunaga, Yukiko, Shiseido Res.Ct.Kanazama Hakkei, Yokohama-shi, Kanagawa 236-0004 (JP); Tsuda, Takanari, Shiseido Res. Ctr. Shin-Yokohama, Yokohama-shi, Kanagawa 224-8558 (JP); Aoyama, Yukari, Shiseido Res. Ctr. Shin-Yokohama, Yokohama-shi, Kanagawa 224-8558 (JP); Koga, Nobuyoshi, Shiseido Res. Ctr. Shin-Yokohama, Yokohama-shi, Kanagawa 224-8558 (JP)
(74) Representative: Santarelli

(56) References cited:
- EP-A- 0 473 502
- EP-A- 1 066 816
- EP-A- 1 163 900
- EP-A- 1 180 371
- EP-A- 1 184 040
- WO-A-94/22470
- PATENT ABSTRACTS OF JAPAN vol. 1996, no. 06, 28 June 1996 (1996-06-28) & JP 08 053336 A (SHISEIDO CO.), 27 February 1996 (1996-02-27)
- PATENT ABSTRACTS OF JAPAN vol. 1999, no. 12, 29 October 1999 (1999-10-29) & JP 11 193212 A (SHISEIDO CO.), 21 July 1999 (1999-07-21)
- PATENT ABSTRACTS OF JAPAN vol. 13, no. 308 (C-617), 14 July 1989 (1989-07-14) & JP 01 093509 A (SHISEIDO CO.), 12 April 1989 (1989-04-12)
- DATABASE WPI Week 199002 Derwent Publications Ltd., London, GB; AN 1990-012965 XP002375940 & JP 01 294635 A (ROHMAN KOGYO KK) 28 November 1989 (1989-11-28)
- DATABASE WPI Week 199929 Derwent Publications Ltd., London, GB; AN 1999-341597 XP002375941 & JP 11 124325 A (NONOGAWA SHOJI KK) 11 May 1999 (1999-05-11)
- DATABASE WPI Week 199953 Derwent Publications Ltd., London, GB; AN 1999-615515 XP002375942 & JP 11 269029 A (SHISEIDO CO.) 5 October 1999 (1999-10-05)

## Description

### Field of the Invention

The present invention relates to an artificial skin production method.

### Prior Art

In the fields of cosmetics and dermatology, a number of various means have been proposed and attempted for alleviation or treatment of skin damage caused by effects of the external environment, including sunlight rays, as well as aging. The major changes in skin that occur with aging are, for example, wrinkle formation, hardening and elasticity loss.

Attention has become focused on the causes of such changes, which include hypofunction of collagen and elastic fibers composed of collagen, elastin and glucosaminoglycans in the dermis. To date, the use of hydroxycarboxylic acids (e.g., Japanese Patent Publication No. 253339) and the use of lysophospholipids (e.g., Japanese Unexamined Patent Publication (KOKAI) No. 8-67621) have been investigated as means for preventing and repairing such skin alterations.

The former publication suggests that cornification or wrinkle formation can be inhibited by preventing loss of collagen fibers. The latter publication, on the other hand, suggests that lysophospholipids have a moisturizing effect in the dermis through accelerating the production of glucosaminoglycans (specifically, hyaluronic acid) in human fibroblasts.

In general terms, skin consists of a horny layer, epidermis, basement membrane and dermis. Type IV and Type VII collagens are components of the epidermal basement membrane responsible for binding the epidermis to the dermis. Type IV collagen is the major component of the lamina densa structure forming the skeleton of the epidermal basement membrane. Type VII collagen is the major component of the anchoring fibrils that bind the basement membrane to the dermis.

It has been observed that the level of expression of Type IV collagen in the epidermal basement membrane decreases with aging (Vazquez F. et al., Maturitas 1996, 25:209-215), while some reports indicate that Type VII collagen production on the protein level and mRNA level also decreases in skin fibroblasts taken from elderly persons compared to skin fibroblasts from younger individuals (Chen et al., J. Invest. Dermatol., 102:205-209, 1994). Also, reduction of the anchoring fibrils composed of Type VII collagen have been reported to occur with physiological aging and photoaging of normal skin (Tsuji, T., Nippi Kaishi 105:963-975, 1995; Tidman et al., J. Invest. Dermatol., 83:448-453, 1984). Thus, acceleration of the formations of Type IV collagen that forms the basement membrane skeleton, anchoring fibrils that bind the epidermis to dermis, as well as Type VII collagen which is the major component of the anchoring fibrils, are deemed as important factors for maintaining healthy and youthful skin.

The most powerful factor in the external environment which affects skin aging is ultraviolet rays included in sunlight, which have been clearly established as an aging-accelerating factor and are known to induce skin changes, so-called photoaging, characterized by deep wrinkles (Scharffetter-Kochanek, Advance in Pharmacology, 1997, 58, 639-655). Ultraviolet rays have various effects on the skin, which include damage to genetic DNA, induced production of active oxygen and, as recently demonstrated, induced production of matrix metalloproteases (Fischer et al., Nature, 1996, 379, 335-339).

Due to the multipotent nature of ultraviolet rays, the mechanisms by which ultraviolet ray-induced photoaging occurs have not yet been adequately elucidated. Experiments involving continuous irradiation of hairless mice with ultraviolet rays at an energy dose which does not cause erythema have demonstrated that deep wrinkles are formed in the mouse dorsal skin corresponding to human photoaged skin, and such mouse models have been used to evaluate substances that can affect wrinkling (Moloney et al., Photochem. Photobiol., 1992, 56, 495-504). However, the mechanisms of wrinkle formation have not yet been adequately elucidated.

On the other hand, Koivulkangas et al. have reported that the activity of the basement membrane catabolic enzyme gelatinase increases in ultraviolet-irradiated skin (Acta Derm. Venereol. 1994, 74, 279-282). Also, Marschall et al. have reported an increase in expression of urokinase (plasminogen activator (uPA)) and its receptor, uPA receptor, in epidermal cells subjected to ultraviolet irradiation (J. Invest. Dermatol. 1999, 113, 69-76), suggesting that supplement of blood plasma-derived plasminogen activates plasminogen surrounding keratinocytes and increases plasmin activity at sites of ultraviolet ray exposure. It has also been reported that the basement membrane exhibits structural changes in sunlight-exposed sites of skin, and particularly extensive multilayering is frequently observed (Lavker, J. Invest. Dermat., 1979, 73, 59-66). This suggests that ultraviolet rays included in sunlight affect the basement membrane structure by increasing production of catabolic enzymes in the skin basement membrane.

Damage to the epidermal basement membrane not only accelerates skin aging but also impedes the daily turnover of epidermis, eliciting further skin damage. Rapid repair of the structure of damaged skin basement membrane is therefore important not only for preventing skin aging, but also as a part of routine skin care.

Specific means for accelerating repair of the basement membrane have been investigated using artificial skin models. As exemplified method for producing an artificial skin model includes culturing normal human epidermal keratinocytes on human fibroblast-containing contracted Type I collagen gel, which has a dermis-like structure, to form an epidermal layer. In this method, a defective basement membrane will be formed between the dermal-imitating collagen gel and the epidermal-imitating layer, and it can therefore be used to evaluate substances that accelerate repair or regeneration of damaged skin basement membrane structure. By using such an artificial skin, it was discovered that acceleration of the regeneration of the skin basement membrane structure can be accomplished by applying a matrix metalloprotease inhibitor, or both the matrix metalloprotease inhibitor and a matrix protein production promoter (Japanese Unexamined Patent Publication (KOKAI) No. 2001-269398). However, it has been a problem that the structure of the regenerated lamina densa is underdeveloped in comparison to normal skin, and the depositing of Type VII collagen is also inadequate.

Artificial skin is important as a substitute for biological skin which has been damaged by any cause, or as an experimental material for testing the effects of skin medicines and cosmetics, or for drug testing, and for all such uses, it is desirable for the artificial skin to have a structure resembling as closely as possible the natural skin structure.

It is an object of the present invention to provide a new method for achieving adequate formation of basement membrane in artificial skin structures.

### Disclosure of Invention

The present inventors have conducted diligent research directed toward solving the problems associated with accelerating repair and regeneration of the skin basement membrane structure and with production of artificial skin having a satisfactory basement membrane structure, and as a result we have completed the present invention upon finding that the basement membrane formation-accelerating effect of matrix metalloprotease inhibitors is notably enhanced by substances that inhibit serine proteases and by substances that increase production of Type IV and Type VII collagen or laminin 5, which are major components of the epidermal basement membrane.

The invention provides as a means for producing artificial skin having an adequately formed basement membrane, an artificial skin-forming medium which comprises one or more serine protease inhibitors, one or more substances that increase production of the extracellular matrix components, for example, the major epidermal basement membrane component Type IV or Type VII collagen or laminin 5, and/or one or more matrix metalloprotease inhibitors, as the active ingredients, either alone or in admixture, as well as a method for producing the medium.

### Brief Description of Drawings

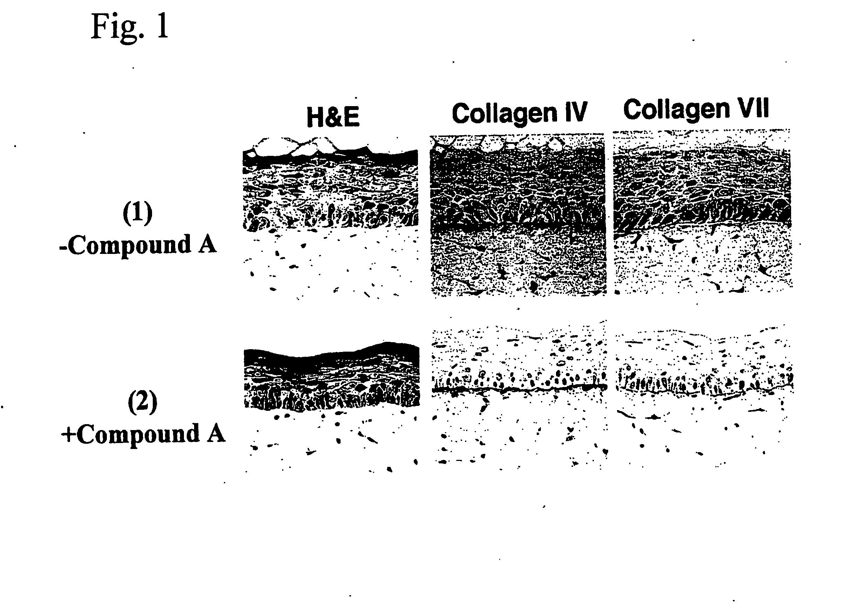

Fig. 1 is a cross-sectional view of artificial skin showing a comparison of culturing to form artificial skin without addition (1) and with addition (2) of the matrix metalloprotease inhibitor compound A to the medium.

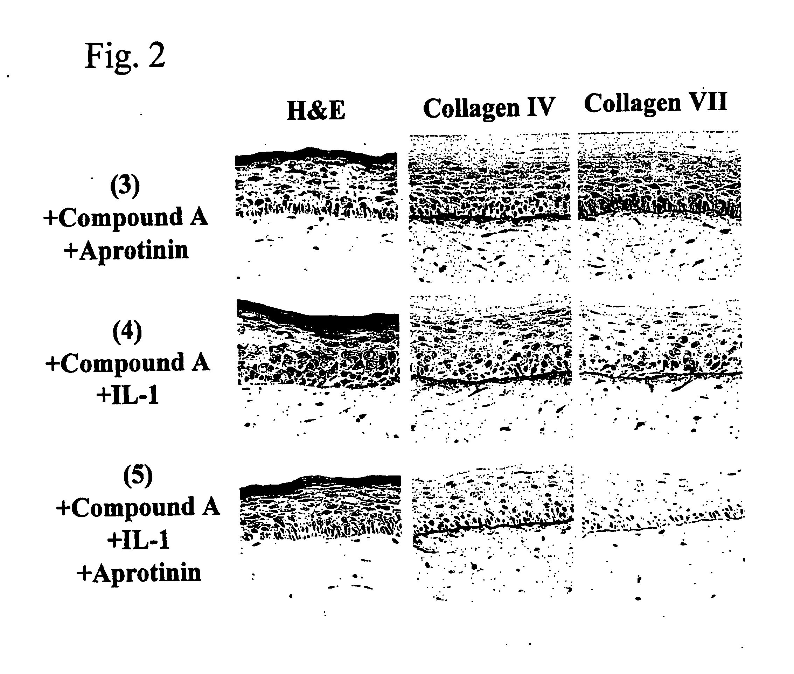

Fig. 2 is a cross-sectional view of artificial skin showing a comparison of culturing to form artificial skin with addition of 10 µM of compound A and 10 µg/ml of aprotinin (3), 10 µM of compound A and 1 ng/ml of interleukin-1β (4), and 10 µM of compound A, 1 ng/ml of interleukin-1β and 10 µg/ml of aprotinin (5) to the medium.

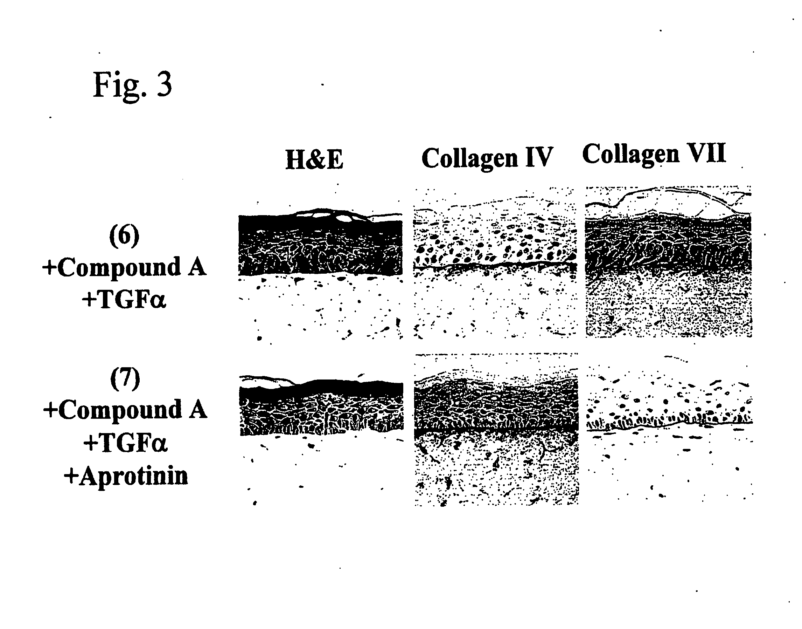

Fig. 3 is a cross-sectional view of artificial skin showing a comparison of culturing to form artificial skin with addition of 10 µM of compound A and 1 ng/ml of TGF-α (6), and 10 µM of compound A, 1 ng/ml of TGF-α and 10 µg/ml of aprotinin (7) to the medium.

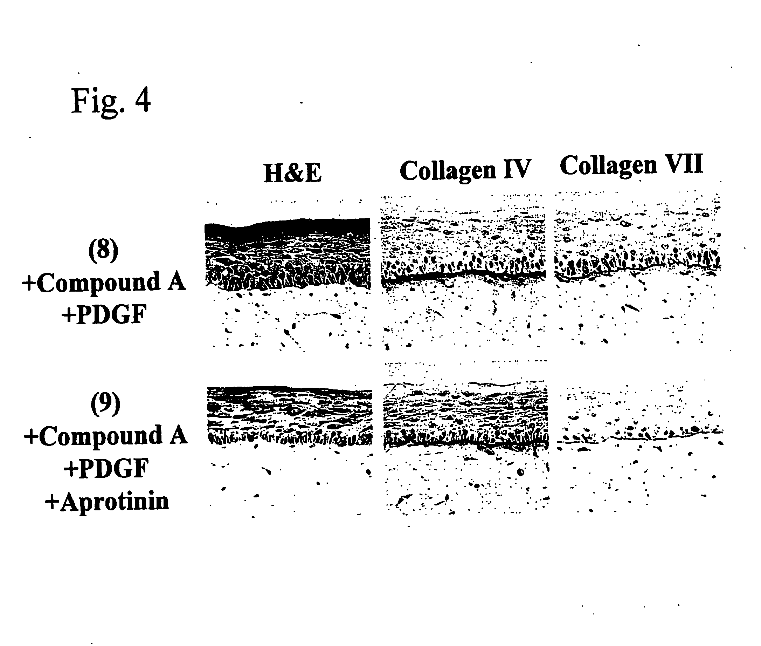

Fig. 4 is a cross-sectional view of artificial skin showing a comparison of culturing to form artificial skin with addition of 10 µM of compound A and 100 ng/ml of PDGF (8) and 10 µM of compound A, 100 ng/ml of PDGF and 10 µg/ml of aprotinin (9) to the medium.

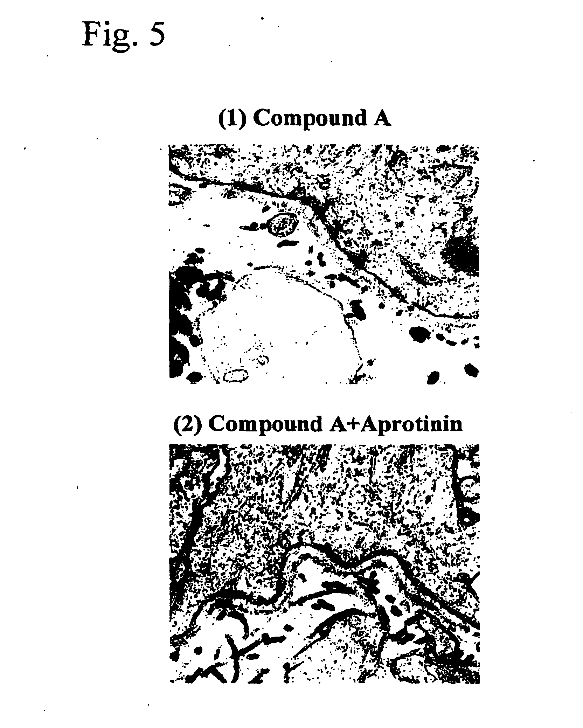

Fig. 5 is a transmission electron microscope photograph of artificial skin showing a comparison of culturing to form artificial skin with addition of the matrix metalloprotease inhibitor compound A alone (1) and with further addition of the serine protease inhibitor aprotinin (2) to the medium.

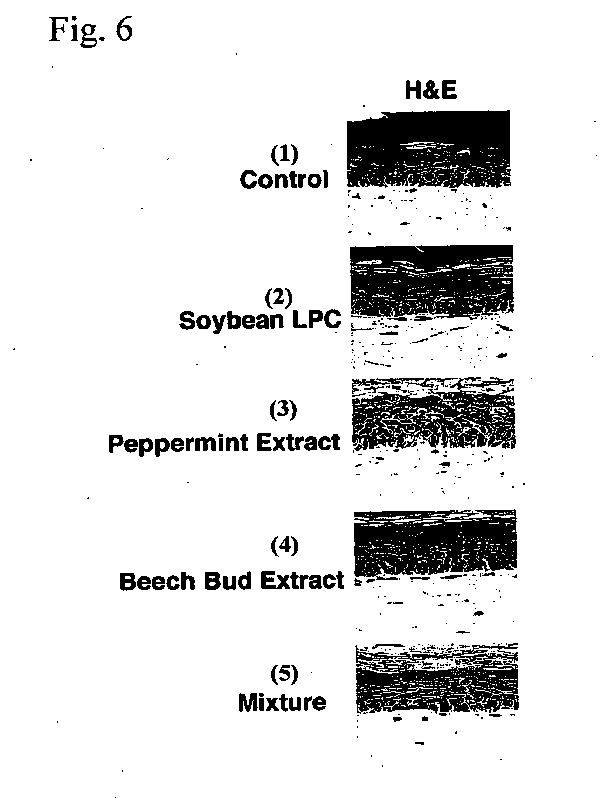

Fig. 6 is a hematoxylin and eosin stained cross-sectional view of artificial skin showing a comparison of culturing to form artificial skin with (1) no addition (control), and addition of (2) soybean lysolecithin, (3) peppermint extract, (4) beech bud extract or (5) soybean lysolecithin + peppermint extract + beech bud extract to the medium.

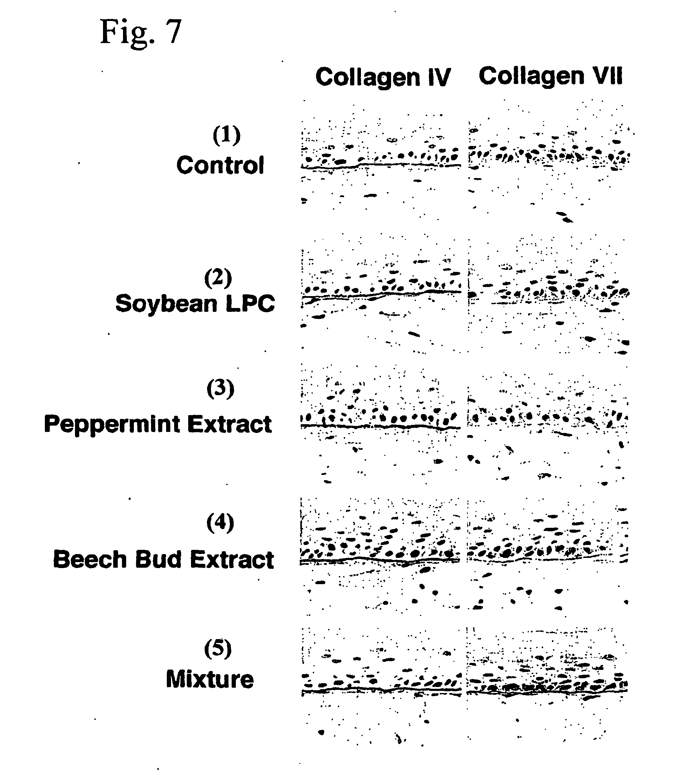

Fig. 7 is an immunostained cross-sectional view of artificial skin showing a comparison of culturing to form artificial skin with (1) no addition (control), and addition of (2) soybean lysolecithin, (3) peppermint extract, (4) beech bud extract or (5) soybean lysolecithin + peppermint extract + beech bud extract to the medium.

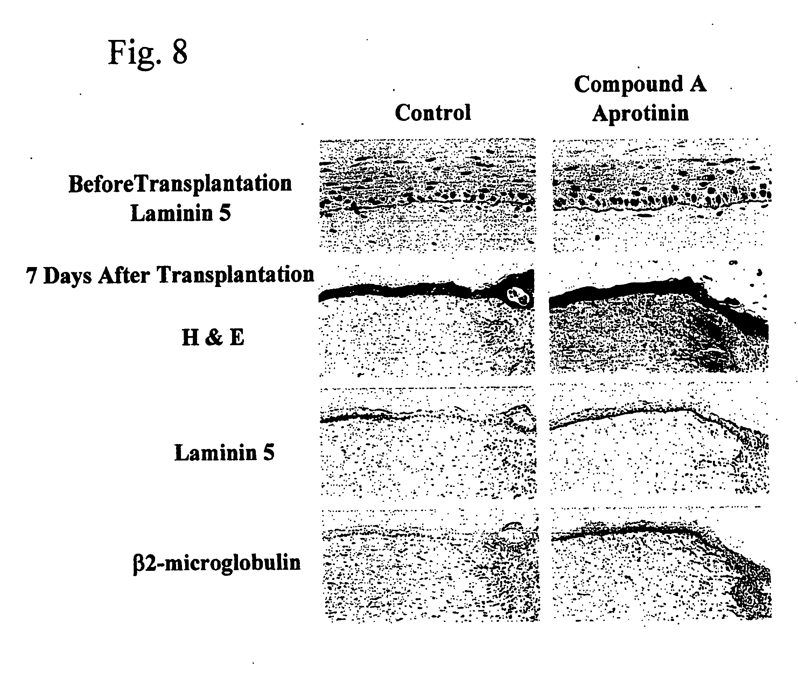

Fig. 8 is a cross-sectional tissue image of artificial skin transplanted into nude mice and collected at day 7 following the transplantation, where the artificial skin had been cultured with no additives or in the presence of the matrix-metalloprotease inhibitor compound A (10 µM) and the serine protease inhibitor aprotinin (10 µg/ml). It shows tissue images of the artificial skin before transplantation (laminin 5 staining) and the adhered artificial skin (hematoxylin and eosin staining, laminin staining, as well as β2-microglobulin staining).

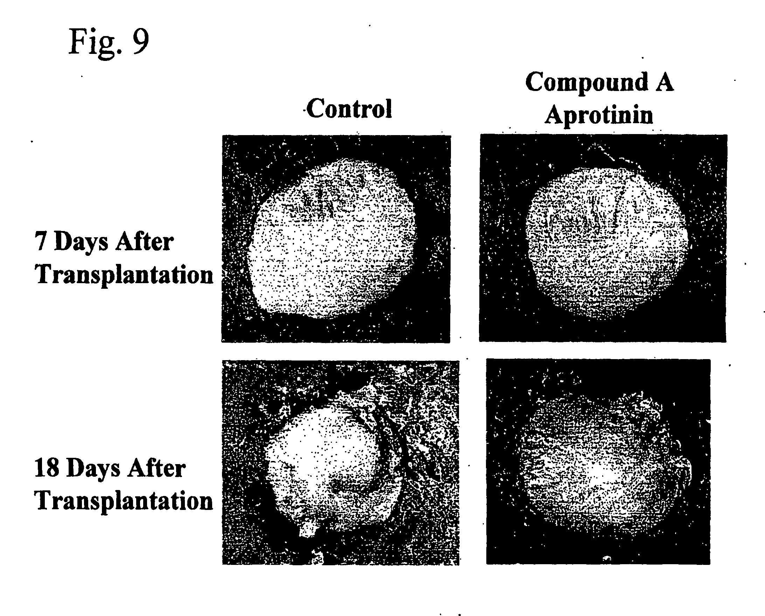

Fig. 9 is a photograph of the outer appearance of artificial skin transplanted into nude mice and collected at days 7 and 18 following the transplantation, where the artificial skin had been cultured with no additive or in the presence of the matrix metalloprotease inhibitor compound A (10 µM) and the serine protease inhibitor aprotinin (10 µg/ml).

### Best Mode for Carrying Out the Invention

### Serine protease inhibitors

There are no particular restrictions on the serine protease-inhibiting substances to be used for the invention so long as they are substances which exhibit such inhibitory activity. Examples of serine proteases include plasmin, urokinase and the like. Thus, the serine protease-inhibiting substances may be selected from among substances that inhibit plasmin, urokinase and the like.

As specific examples of serine protease-inhibiting substances, there may be mentioned aprotinin, tranexamic acid and ε-aminocaproic acid, or the like.

Specific examples of serine protease inhibitors that may be used according to the invention include various plant extracts that exhibit serine protease-inhibiting activity, and their purified products. As such plants there may be mentioned *Calophyllum brasiliense* Cambess., *Myrcia sphaerocarpa* DC, *Hyptis crenata* Pohl ex Benth., *C*. *rotundus* L, *E. sylvestris var. ellipticus* and *E*. *decipiens Helmsl.;* plants belonging to the *Rosaceae* family, and especially plants belonging to the genera *Rosa, Rubus*, *Filipendula* and *Crataegus* of the *Rosaceae* family; plants belonging to the family *Paeoniaceae*, genus *Paeonia;* plants belonging to the family *Moraceae*, genus *Humulus,* plants belonging to the family *Compositae,* genus *Arnica* and the family *Compositae*, genus *Anthemis*, plants belonging to the family *Pyrolaceae,* plants belonging to the family *Hypericaceae* such as *Hypericium erectum T.* and *Hypericium perforatum*, plants belonging to the family *Labiatae*, genus *Mentha* such as *Mentha piperata* L. and *Mentha viridis* L.; plants belonging to *Compositae Carthamus tinctorius* L., *Geraniaceae Geranium thunbergii* Siebold et zuccarini and *Vitaceae Vitis*; plants belonging to the family *Betulaceae* such as *Betula alba* L., *Betula lutea* L. and *Betula pendula* Roth.; plants belonging to the family *Rosaceae,* genus Prunus, which are apricots (*P*. *armeniaca* L., *P ansu* Komar, *P. mandshurica* Koehne, *P*. *sibirica* L.); and plants which grow in dry prairies or grasslands, such as zapote, que shar, sano-sano, cora de cavallo, and the like.

Such plant extracts may be obtained from the roots, leaves, stems, flowers, etc. of herbal plants or from the roots, buds, bark, fruit, leaves, flowers, etc. of woody plants.

The extracts derived from these plants may be obtained by drying the plant material, if necessary, and further slicing or pulverizing it, if necessary, and then using cold water, warm water or boiling/hot water to prepare aqueous extracts, while organic solvents such as methanol, ethanol, 1,3-butanediol and ether may also be used therewith at ambient temperature or with heating.

### Extracellular matrix protein production accelerators

The extracellular matrix protein production accelerators to be used for the invention are substances which accelerate the production of such proteins.

As matrix proteins according to the present invention, there may be mentioned the basement membrane components laminin, Type VII collagen, perlecan, nidogen and the like, and especially laminin 5, Type IV collagen and Type VII collagen, which are distinctive components of the skin basement membrane.

As specific examples of laminin 5 production accelerators, there may be mentioned transforming growth factor-α, transforming growth factor-β1, transforming growth factor-β2, transforming growth factor-β3, epidermal growth factor, and the like.

Various plant extracts and their purified forms may also be used. As such plants, there may be mentioned glycyrrhiza, blackberry lily, *Alstonia scholaris*, Tinospora crispa, fenugreek, *Papaveraceae Bocconia palo amarillo, Leguminosae Psophocarpus tetragonolobus, Leguminosae Cassia retama, Gentianaceae Erythraea chilensis* (canchalagua), soybean, pueraria root, cammock, melilot, sprouts, red bean, and the like. These plant extracts may be obtained from the roots, leaves, stems, flowers, etc. of herbal plants or from the roots, buds, bark, fruit, leaves, flowers, etc. of woody plants.

The extracts from these plants may be obtained by drying the plant material, if necessary, and further slicing or pulverizing it, if necessary, and then using cold water, warm water or boiling/hot water to prepare aqueous extracts, while organic solvents such as methanol, ethanol, 1,3-butanediol and ether may also be used therewith at ordinary temperature or with heating. Purified extracts from such plants include soybean lysolecithin, soybean saponin fragments, soybean lecithin fragments, and the like.

As specific examples of Type IV collagen production accelerators there may be mentioned tumor necrosis factor-α, transforming growth factor-β1, transforming growth factor-β2, transforming growth factor-β3, epidermal growth factor, interleukin-1α (IL-1α), interleukin-1β (IL-1β), platelet-derived growth factor, and the like.

Various animal-derived materials may also be used. For example, there may be mentioned bromois milk prepared from milk casein, EM protein L prepared by hydrolysis of chicken egg shell membrane, and the like.

Various plant-derived extracts and their purified products may also be used. As such plants there may be mentioned plants belonging to the family *Apocynaceae* such as pule (*Alstonia scholars*), trees belonging to the family *Fagaceae* such as *Fagus sylvatica*, plants of the family *Leguminosae* such as *Pueraria lobata* Ohwi, plants of the family *Araliaceae* such as *Hedera helix,* and plants such as orchids and the like. Such plant extracts may be obtained from the roots, leaves, stems, flowers, etc. of herbal plants or from the roots, buds, bark, fruit, leaves, flowers, etc. of woody plants.

The extracts from these plants may be obtained by drying the plant material, if necessary, and further slicing or pulverizing it, if necessary, and then using cold water, warm water or boiling/hot water to prepare aqueous extracts, while organic solvents such as methanol, ethanol, 1,3-butanediol and ether may also be used therewith at ambient temperature or with heating.

As specific examples of Type VII collagen production accelerators, there may be mentioned tumor necrosis factor-α, transforming growth factor-β1, transforming growth factor-β2, transforming growth factor-β3, epidermal growth factor, interleukin-1α (IL-1α), interleukin-1β, platelet-derived growth factor, and the like.

Various animal-derived substances may also be used. For example, there may be mentioned bromois milk prepared from milk casein, EM protein L prepared by hydrolysis of chicken egg shell membrane, and the like.

Various plant-derived extracts and their purified products may also be used. As such plants there may be mentioned plants belonging to the family *Apocynaceae* such as pule (*Alstonia scholaris*), trees belonging to the family *Fagaceae* such as *Fagus sylvatica,* plants of the family *Leguminosae* such as *Pueraria lobata* Ohwi, plants of the family *Araliaceae* such as *Hedera helix,* and plants such as orchids and the like. Such plant extracts may be obtained from the roots, leaves, stems, flowers, etc. of herbal plants or from the roots, buds, bark, fruit, leaves, flowers, etc. of woody plants.

The extracts from these plants may be obtained by drying the plant material, if necessary, and further slicing or pulverizing it, if necessary, and then using cold water, warm water or boiling/hot water to prepare aqueous extracts, while organic solvents such as methanol, ethanol, 1,3-butanediol and ether may also be used therewith at ambient temperature or with heating.

### Matrix metalloprotease inhibitors

There are no particular restrictions on the matrix metalloprotease inhibitors that may be used for the present invention, so long as they are substances which exhibit such inhibitory activity. Examples of matrix metalloproteases include gelatinase, collagenase, stromelysin, matrilysin, and the like. Thus, the matrix metalloprotease inhibitors may be selected from among substances that inhibit gelatinase, collagenase, stromelysin, matrilysin, and the like.

As specific examples of matrix metalloprotease inhibitors, there may be mentioned N-hydroxy-2-[[(4-methoxyphenyl) sulfonyl]-3-picolyl]amino)]-3-methyl butaneamide hydrochloride (hereinafter referred to as "Compound A") (J. Med. Chem. 1997, 40, p.2525-2532), MMP-inhibitor (p-NH₂-Bz-Gly-Pro-D-Leu-Ala-NHOH)(FN-437)(BBRC, 1994, 199, 1442-1446), etc.

Specific examples of matrix metalloprotease inhibitors according to the present invention include various plant extracts and their purified products. As such plants there may be mentioned *Thymus serpyllum* L., *Valeriana fauriei* Briquet and other plants of the family *Valerianaceae, Diospyros kaki* Thunberg (family *Ebenaceae*), *Astragalus sinicus* Linne (family *Leguminosae*), *Crataegus cuneata* Siebold et Zuccarini (family *Rosaceae*), *Paeonia suffruticosa* Andrews (Poeonia montan Sims) (family *Paconiaceae*), *Thea sinensis* Linne var. assamica Pierre, (family *Theaceae*), *Eucalyptus globulus* Labillardiere and other plants of the family *Myrtaceae, Potentilla tormentilla* Schrank (family *Rosaceae*), *Tilia cordata* Mill., *Tilia platyphyllus* Scop., *Tilia europaea* Linne (family *Tiliaceae*), *Betula alba* Linne (*family Betulaceae*), *Origanum majorana* L., *Uncaria gambir* Roxburgh (family *Rubiaceae*), *Juglans regia* Linne var. sinensis De Candolie or other plants of the family *Juglandaceae, Sophora flavescens* Aiton (family *Leguminosae*), Sanguisorba *officinalis* Linne (family *Rosaceae*), *Hypericum perforatum* Linne or *Hypericum erectum* Thunberg (family Guttiferae), *Thea sinensis* Linne (family *Theaceae*), *Curcuma longa* L (family *Zingiberaceae*), the turmeric purified extract curcumin, *Symplocos racemosa, Cyperus rotundus, Cyperus scariosus, Gaultheria fragrantissima, Acacia fornensia, Terminalia chebula*, *Ficus bengalensis* (Banyan tree), *Cassia fistula* Linn., *Lyonia ovalifolia, Calophyllum inophyllum, Ficus religiosa,* and the like.

Such plant extracts may be obtained from the roots, leaves, stems, flowers, etc. of herbal plants or from the roots, buds, bark, fruit, leaves, flowers, etc. of woody plants.

The extracts from these plants may be obtained by drying the plant material, if necessary, and further slicing or pulverizing it, if necessary, and then using cold water, warm water or boiling/hot water to prepare aqueous extracts, while organic solvents such as methanol, ethanol, 1,3-butanediol and ether may also be used therewith at ambient temperature or with heating.

### Artificial skin

The basic medium used for production of artificial skin according to the present invention may be any medium which is conventionally used for producting artificial skin, and such media include Dulbecco's modified Eagle medium (DMEM) containing 10% fetal bovine serum; DMEM-Ham's F12 (3:1) containing 10% fetal bovine serum, 5 µg/ml transferrin, 5 µg/ml insulin, 2 nM tri-iodothyronine, 0.1 nM cholera toxin and 0.4 µg/ml hydrocortisone; and a 1:1 mixture of keratinocyte growth medium (KGM) and DMEM containing 10% fetal bovine serum. The amount of serine protease inhibitor added to such basic media will differ depending on the type, but will generally be from about 1 ng/L to 1 g/L. The amount of the matrix protein production accelerator added to the basic medium will be from about 1 ng/L to 1 g/L, and the amount of matrix metalloprotease inhibitor added will be from about 1 nmol/L to 10⁻² mole/L.

For production of artificial skin according to the present invention, human fibroblast-containing contracted Type I collagen gel is first placed on a wire mesh. The human fibroblast-containing contracted Type I collagen gel may be prepared, for example, in the following manner. A fibroblast-suspended collagen solution is prepared while cooling on ice, and then the collagen is gelled in a Petri dish. Next, the gel is peeled from the Petri dish walls and the collagen gel is contracted in a CO₂ incubator.

Epidermal cells, such as normal human epidermal keratinocytes, are then cultured on the collagen gel to form an epidermis. The epidermal layer may be formed by culturing skin cells in the following manner. The contracted collagen gel is placed on a wire mesh and a glass ring is placed over the gel. A human foreskin-derived epidermal keratinocyte suspension is loaded into the glass ring while avoiding leakage. The keratinocytes are allowed to adhere in a CO₂ incubator, and the ring is removed. The medium is filled in up to the border of the epidermal layer, and culturing is continued while exposing the epidermal layer to the air, to form a horny layer.

According to this method it is possible to obtain natural skin-like artificial skin having an adequate layer of basement membrane components deposited between a dermal layer composed of fibroblast-containing Type I contracted collagen gel and an epidermal layer.

### Example 1 Production of artificial skin using matrix metalloprotease inhibitors, extracellular matrix production accelerators (cytokines) and serine protease inhibitors

Collagen gel was obtained by preparing 10 ml of a human dermal fibroblasts (0.3-1 x 10⁵ cell/ml)-suspended collagen solution (using I-AC collagen manufactured by Koken Co., Ltd.) on ice, followed by gelling the collagen in a 60 mm Petri dish at 37°C. The gel was then peeled from the bottom of the dish, the collagen gel was placed on metal, and a glass ring (12 mm inner diameter) was placed over the gel. A 0.4 ml portion of a human foreskin-derived epidermal keratinocyte (1 x 10⁶/ml) suspension (KGM-DMEM (1:1) mixed medium containing 5% fetal bovine serum) was poured into the glass ring while avoiding leakage. The keratinocytes were allowed to adhere in a CO₂ incubator overnight, and the ring was removed on the following day. The medium was filled in up to the border of the epidermal layer, and culturing was continued while exposing the epidermal layer to the air, to prepare a multilayered skin model with epidermis having a horny layer.

From the 4th day after seeding the epidermal cells, the medium was replaced with medium containing (1) no compound (-compound A), (2) 10 uM of compound A (+compound A), (3) 10 µM of compound A and 10 µg/ml of aprotinin (+compound A + aprotinin), (4) 10 µM of compound A and 1 ng/ml of interleukin-1β (+compound A + IL-1), (5) 10 µM of compound A, 1 ng/ml of interleukin-1β and 10 µg/ml of aprotinin (+compound A + IL-1 + aprotinin), (6) 10 µM of compound A and 1 ng/ml of TFG-α (+compound A + TGFα), (7) 10 µM of compound A, 1 ng/ml of TGF-α and 10 µg/ml aprotinin (+compound A, +TGFα + aprotinin), (8) 10 µM of compound A and 100 ng/ml PDGF (+compound A + PDGF) and (9) 10 µm of compound A, 100 ng/ml PDGF and 10 µg/ml of aprotinin (+compound A + PDGF + aprotinin), and every 2-3 days thereafter, the medium was exchanged with a medium containing the same types and same concentrations of matrix metalloprotease, extracellular matrix production accelerator (cytokines) and serine protease inhibitor, after which culturing was performed for 2 weeks.

The formed artificial skin was stained with hematoxylin and eosin (H&E) and immunostaining (using anti-type IV collagen antibody and anti-type VII collagen antibody). The results are shown in Figs. 1 to 4.

Although virtually no staining of the type VII collagen directly under the basal keratinocytes was observed in the control (1), weak but definite staining of type VII collagen was observed when the matrix metalloprotease inhibitor compound A (2) was added. The staining of type VII collagen further increased when the serine protease inhibitor aprotinin (3) was added in addition to the matrix metalloprotease inhibitor compound A. Staining of type VII collagen was particularly accentuated when compound A was accompanied with the matrix protein production-increasing compounds interleukin 1 (IL-1) (4), transforming growth factor-α (TGF-a) (6) and platelet-derived growth factor (PDGF) (8). When the matrix protein production-increasing compounds interleukin 1 (IL-1) (5), transforming growth factor-α (TGF-α) (7) and platelet-derived growth factor (PDGF) (9) were combined with aprotinin and compound A, the staining of type IV and type VII collagen was clearly concentrated directly under the basal keratinocytes, giving a sharp stained image, thus suggesting that each of the molecules probably constructs an appropriate three-dimensional structure.

The same culturing process as described above was carried out using the following plant extracts instead of the aforementioned matrix metalloprotease inhibitor, compound A.

*Thymus serpyllum L., Valeriana fauriei* Briquet and other plants of the family *Valerianaceae, Diospyros kaki* Thunberg (family *Ebenaceae*), *Astragalus sinicus* Linne (family *Leguminosae*), *Crataegus* cuneata Siebold et Zuccarini (family *Rosaceae*), *Paeonia suffruticosa* Andrews (Poeonia montan Sims)(family *Paconiaceae*), *Thea sinensis* Linne var. assamica Pierre, (family *Theaceae*), *Eucalyptus globulus* Labillardiere and other plants of the family *Myrtaceae, Potentilla tormentilla* Schrank (family *Rosaceae*), *Tilia cordata* Mill., *Tilia platyphyllus* Scop., *Tilia europaea* Linne (family *Tiliaceae*), *Betula alba* Linne (family *Betulaceae*), *Origanum majorana* L., *Uncaria gambir* Roxburgh (family *Rubiaceae*), *Juglans regia* Linne var. sinensis De Candolie or other plants of the family *Juglandaceae, Sophora flavescens* Aiton (family *Leguminosae*), *Sanguisorba officinalis* Linne (family *Rosaceae*), *Hypericum perforatum* Linne or *Hypericum erectum* Thunberg (family *Guttiferae*), *Thea sinensis* Linne (family *Theaceae*), *Curcuma longa* L (family *Zingiberaceae*), the turmeric purified extract curcumin, *Symplocos racemosa, Cyperus rotundus, Cyperus scariosus, Gaultheria fragrantissima, Acacia fornensia, Terminalia chebula, Ficus bengalensis* (Banyan tree), *Cassia fistula* Linn., *Lyonia ovalifolia, Calophyllum inophyllum, Ficus religiosa.*

The similar results were obtained as when compound A was used as the matrix metalloprotease.

The same culturing process as described above was carried out using the following plant extracts instead of the aforementioned serine protease inhibitor, aprotinin.

*Calophyllum brasiliense* Cambess., *Myrcia sphaerocarpa* DC, *Hyptis crenata* Pohl ex Benth., *C*. *rotundus L, E. sylvestris var. ellipticus, E. decipiens Helmsl.,* plants belonging to genera *Rosa, Rubus, Filipendula* and *Crataegus* of the *Rosaceae* family; plants belonging to the family *Paeoniaceae,* genus *Paeonia;* plants belonging to the family *Moraceae,* genus *Humulus,* plants belonging to the family *Compositae,* genus *Arnica* and the family *Compositae,* genus *Anthemis,* plants belonging to the family *Pyrolaceae,* plants belonging to the family *Hypericaceae;* plants belonging to *Mentha piperata* L. and *Mentha viridis* L.; plants belonging to *Compositae Carthamus tinctorius L., Geraniaceae Geranium thunbergii* Siebold et Zuccarini and *Vitaceae Vitis;* plants belonging to the family *Betulaceae* such as *Betula alba L., Betula lutea* L. and *Betula pendula* Roth.; plants belonging to the family *Rosaceae,* genus *Prunus,* which are apricots (*P. armeniaca* L., *P ansu* Komar, *P. mandshurica* Koehne, *P*. *sibirica* L.); and zapote, que shar, sano-sano, cora de cavallo.

The similar results were obtained as when aprotinin was used as the serine protease inhibitor.

The same culturing process as described above was carried out using, instead of the matrix protein production accelerators IL-1, TGF-α and PDGF, the following laminin 5 production-accelerating agents: extracts of soybean lysolecithin, glycyrrhiza, blackberry lily, jitanoki, Tinospora crispa, fenugreek, Papaveraceae *Bocconia palo amarillo, Leguminosae Psophocarpus tetragonolobus, Leguminosae Cassia retama, Gentianaceae* Erythraea *chilensis* (canchalagua), soybean, pueraria root, cammock, melilot, sprouts and red bean. The same culturing was also carried out using, as type IV collagen or type VII collagen production-accelerating agents, pule (*Alstonia scholaris),* beech (*Fagus sylvatica*), kudzu vine *Pueraria lobata* Ohwi), and English ivy (*Hedera helix*) extracts, and as a type VII collagen production-accelerating agent, orchid extract. The similar results were obtained as when IL-1, TGF-α and PDGF were used.

A transmission electron microscope was used to observe the basement membrane structure directly under the epidermis after adding the matrix metalloprotease inhibitor compound A (2) or after adding the serine protease inhibitor aprotinin (3) with the matrix metalloprotease inhibitor compound A, and as shown in Fig. 5, compound A induced formation of the basement membrane, while further addition of aprotinin increased the electron density and accentuated the continuity of the basement membrane.

### Example 2 Production of artificial skin using extracellular matrix production accelerators and serine protease inhibitors

The method of Example 1 was repeated, but the culturing process was conducted with the following test substances.
(1) No addition (control);
(2) 30 µg/ml of the laminin 5 production accelerator "soybean lysolecithin" alone (soybean LPC);
(3) 0.5% of the serine protease inhibitor "peppermint extract" alone;
(4) 0.3% of the type IV and type VII collagen production accelerator "beech bud extract" alone;
(5) 30 µg/ml of the laminin 5 production accelerator "soybean lysolecithin", 0.5% of the serine protease inhibitor "peppermint extract" and 0.3% of the type IV and type VII collagen production accelerator "beech bud extract" It (mixture).

The formed artificial skin was stained with hematoxylin and eosin (H&E) staining and immunostaining (using anti-type IV collagen antibody and anti-type VII collagen antibody). The results are shown in Figs. 6 and 7.

In the control (1), the type IV collagen was weakly stained but virtually no staining of the type VII collagen was observed. In contrast, the staining of type IV collagen increased with the laminin 5 production accelerator, soybean lysolecithin (2). The staining of type VII collagen increased with the peppermint extract (3). The production of type IV and type VII collagen increased with the beech bud extract (4). By combining them (5), a clear increase was confirmed in the staining of type VII collagen.

### Example 3

### Artificial skin transplant

Artificial skin was prepared in the same manner as Example 1. Following the loading of epidermal cells, the artificial skins were cultured from three days to one week, in the absence of any additive, i.e. the control artificial skin, or in the presence of the basement membrane ameliorator, matrix metalloprotease inhibitor compound A (10 µl) and the serine protease inhibitor aprotinin (10 µg/ml), after which each of them was transplanted at dorsal skin-resected sites of nude mice. Silicone gauze was placed over each transplant site, and the site was covered with Tegaterm elastic plastic adhesive tape (Sumitomo 3M). 7 days after the transplantation, external observation was carried out, and then the tissues were collected. The collected tissues were fixed with cold acetone, and paraffin sections were prepared in a conventional way. The transplanted artificial skin was stained with hematoxylin and eosin staining (H&E) or immunostained with anti-laminin 5 antibody (laminin 5). The results are shown in Figs. 8 and 9.

Referring to Fig. 8, it can be seen that, by comparing the transplanted artificial skins, in the control group, some of the laminin 5 regions underneath the epidermis are remained unstained, while in the group cultured in the presence of matrix metalloprotease inhibitor compound A and aprotinin, abetter and mostly uniformed laminin 5 staining was resulted. A histological analysis carried out at 7 days after the transplantation showed that, in the control group, gaps at the dermal epidermal junction region in the artificial skin were observed, and it was also found that the stained laminin 5 region was broadened, suggesting a possibility of the damage of the basement membrane. Further, the thickness and the configuration of the epidermis were being inferior. On the contrary, in the additive group, the configuration of the dermal-epidermal junction region was good, and it also showed a continuous staining of laminin 5. Further, the state of the epidermis was better.

Moreover, this difference is distinct when carrying out an observation of the boundary portion between the artificial skin and the true skin. In particular, when transplanting the artificial skin having a satisfactory basement membrane, in comparison with the control group, it could be seen that the transplanted artificial skin shows a good adhesion, and the staining of the human-derived laminin 5 and the staining of the β-microglobulin, which specifically recognizes a human cell, extremely close to the boundary were observed, and the satisfactory skin prevented the penetration of the mouse-derived epidermis. Also, an angiogenesis originated from the boundary portion was promoted. Thus, if transplanting an artificial skin having a satisfactory basement membrane due to the incorporation of the matrix metalloprotease inhibitor and aprotinin, it could be seen that the post-transplantation state would significantly improved.

Comparing the external photographs (Fig. 9), with respect to the state at day 18 after the transplantation, in the artificial skin group having a satisfactory basement membrane due to the incorporation of the matrix metalloprotease-inhibitor and aprotinin, in comparison with the additive-free control group, the contraction caused by the surrounding mouse skin was suppressed, and the formation of the surface horny layer was also significantly improved.

It was thus confirmed that transplantation of skin models with satisfactory basement membranes results in a good adhesiveness of the transplanted tissue, as well as a more attractive maintenance of the transplant sites due to a more satisfactory epidermal and dermal condition.

Serine protease inhibitors and accelerators of production of type IV and/or type VII collagen and laminin 5, which are major epidermal basement membrane constituents, notably enhance the basement membrane formation accelerating effect of matrix metalloprotease inhibitors.

It will be appreciated by those skilled in the art that while the invention has been described above in connection with particular embodiments and examples, the invention is not necessarily so limited and that numerous other embodiments, examples, uses, modifications and departures from the embodiments, examples and use may be made without departing from the inventive scope of this application.

## Claims

1. A method for producing an artificial skin, comprising a step of culturing an artificial skin-forming medium, **characterized by** adding one or more serine protease inhibitors to said artificial skin-forming medium.

2. The method according to claim 1, **characterized by** further adding one or more extracellular matrix protein production accelerators to the artificial skin-forming medium.

3. The method according to claim 2, wherein said extracellular matrix proteins are one or more selected from the group consisting of Type IV collagen, Type VII collagen and laminin 5.

4. The method according to any one of claims 1 to 3, wherein there are further added one or more matrix metalloprotease inhibitors.

5. The method according to any one of claims 1 to 3, wherein said serine protease inhibitor is aprotinin.

6. The method according to any one of claims 1 to 5, wherein said extracellular matrix protein production accelerator is interleukin-1, transforming growth factor-α or platelet-derived growth factor.

## Patentansprüche

1. Verfahren zur Herstellung einer künstlichen Haut, umfassend eine Stufe der Kultivierung eines künstliche Haut bildendes Mediums, **gekennzeichnet durch** die Zugabe einer oder mehrerer Serinprotease-Inhibitoren zu dem künstliche Haut bildenden Medium.

2. Verfahren nach Anspruch 1, **gekennzeichnet durch** die weitere Zugabe einer oder mehrerer Beschleuniger der extrazellulären Matrixproteinproduktion zu dem künstliche Haut bildenden Medium.

3. Verfahren nach Anspruch 2, wobei die extrazellulären Matrixproteine eines oder mehrere, ausgewählt aus der Gruppe, bestehend aus Typ IV Kollagen, Typ VII Kollagen und Laminin 5, sind.

4. Verfahren nach einem der Ansprüche 1 bis 3, wobei ferner ein oder mehrere Matrixmetallprotease-Inhibitoren zugefügt werden.

5. Verfahren nach einem der Ansprüche 1 bis 3, wobei der Serinprotease-Inhibitor Aprotinin ist.

6. Verfahren nach einem der Ansprüche 1 bis 5, wobei der Beschleuniger der extrazellulären Matrixproteinproduktion Interleukin-1, transformierender Wachstumsfaktor-α oder von Blutplättchen abgeleiteter Wachstumsfaktor ist.

## Revendications

1. Procédé pour la production d'une peau artificielle, comprenant une étape de culture d'un milieu de formation de peau artificielle, **caractérisé par** l'addition d'un ou plusieurs inhibiteurs de sérine-protéases audit milieu de formation de peau artificielle.

2. Procédé suivant la revendication 1, **caractérisé par** l'addition supplémentaire d'un ou plusieurs accélérateurs de production de protéines de matrice extracellulaire au milieu de formation de peau artificielle.

3. Procédé suivant la revendication 2, dans lequel lesdites protéines de matrice extracellulaire consistent en une ou plusieurs protéines choisies dans le groupe consistant en le collagène de type IV, le collagène du type VII et la laminine 5.

4. Procédé suivant l'une quelconque des revendications 1 à 3, dans lequel sont ajoutés en outre un ou plusieurs inhibiteurs de métalloprotéases de matrice.

5. Procédé suivant l'une quelconque des revendications 1 à 3, dans lequel ledit inhibiteur de sérine-protéases est l'aprotinine.

6. Procédé suivant l'une quelconque des revendications 1 à 5, dans lequel ledit accélérateur de production de protéines de matrice extracellulaire est l'interleukine-1, le facteur de croissance transformant α ou le facteur de croissance dérivé des plaquettes.
